Europäisches Patentamt

(19) European Patent Office

Office européen des brevets

(11) **EP 0 558 415 B1**

## (12) FASCICULE DE BREVET EUROPEEN

(45) Date de publication et mention
de la délivrance du brevet:
**22.01.1997 Bulletin 1997/04**

(51) Int. Cl.$^6$: **C07C 51/25**, C07C 17/156,
C07C 51/215, C07B 33/00,
C07B 41/08

(21) Numéro de dépôt: **93400483.9**

(22) Date de dépôt: **25.02.1993**

(54) **Procédé pour améliorer une réaction d'oxydation ménagée**

Verfahren zur Verbesserung einer schonenden Oxidation

Method of improving a smooth oxidation reaction

(84) Etats contractants désignés:
**BE DE ES IT NL**

(30) Priorité: **27.02.1992 FR 9202277**

(43) Date de publication de la demande:
**01.09.1993 Bulletin 1993/35**

(73) Titulaire: **L'AIR LIQUIDE, SOCIETE ANONYME POUR
L'ETUDE ET L'EXPLOITATION DES PROCEDES
GEORGES CLAUDE
F-75321 Paris Cédex 07 (FR)**

(72) Inventeurs:
  • **Arpentinier, Phillipe
    F-75008 Paris (FR)**
  • **Koenig, Jacques
    F-78000 Versailles (FR)**
  • **Torre, Yves
    F-78470 Cressely (FR)**

(74) Mandataire: **Portal, Gérard et al
    Cabinet Beau de Loménie
    158, rue de l'Université
    75340 Paris Cédex 07 (FR)**

(56) Documents cités:
    **EP-A- 0 029 317        DE-B- 1 254 137
    FR-A- 2 281 361**

Printed by Rank Xerox (UK) Business Services
2.13.13/3.4

**Description**

La présente invention concerne un procédé pour améliorer la sélectivité d'une réaction d'oxydation ménagée entre au moins un composé organique et de l'oxyène en vue de l'obtention d'un produit final et améliorer soit la capacité soit le rendement d'une unité de production où cette réaction d'oxydation ménagée est mise en oeuvre.

La plupart des réactions d'oxydation mises en oeuvre dans l'industrie chimique suivent le schéma ci-après :

$$\text{REACTIF(S)} + O_2 \rightarrow \text{PRODUIT(S)} \qquad \text{Oxydation ménagée} \qquad\qquad \text{(I)}$$

$$\text{REACTIF(S)} + O_2 \rightarrow CO_2 + H_2O \qquad \text{Oxydation complète} \qquad\qquad \text{(II)}$$

$$\text{PRODUIT(S)} + O_2 \rightarrow CO_2 + H_2O \qquad\qquad\qquad\qquad \text{(III)}$$

$$\text{REACTIFS} + O_2 \rightarrow \text{COPRODUITS} \qquad \text{Réactions secondaires} \qquad\qquad \text{(IV)}$$

$$\text{COPRODUITS} + O_2 \rightarrow CO_2 + H_2O \qquad\qquad\qquad\qquad \text{(V)}$$

La réaction (I) d'oxydation ménagée, est celle conduisant au produit recherché et qu'il convient donc de favoriser par rapport aux autres réactions.

Les réactions (I) et (II) sont généralement prépondérantes, les autres étant souvent nettement moins importantes.

Il faut noter que la réaction (II) consistant en l'oxydation totale des réactifs en $CO_2$ et $H_2O$, est nettement plus exothermique que la réaction principale (I).

Ce type de réactions d'oxydation, dans des conditions industriellles, utilise habituellement l'air comme source d'oxygène, l'azote de l'air étant inerte vis à vis des réactifs mis en oeuvre.

La sélectivité du procédé en produit recherché est généralement faible vis-à-vis de la sélectivité maximale théorique. Cette sélectivité relativement basse s'explique par la forte contribution des réactions annexes, en particulier la réaction (II), avec le dégagement de chaleur correspondant, ce qui entraîne des problèmes technologiques d'évacuation de cette chaleur et limite le taux de conversion par passe.

On a décrit dans le brevet EP-A-0029317 un procédé de préparation d'anhydride maléique par oxydation d'un hydrocarbure à chaîne droite en $C_4$ par l'oxygène en présence éventuellement d'un gaz inerte.

Ce même document propose d'améliorer la sélectivité de la réaction en réalisant l'oxydation en présence de dioxyde de carbone.

On a également décrit dans le brevet français FR-A-2281361 un procédé de préparation d'oxyde d'éthylène par oxydation ménagée d'éthylène en présence d'un tiers gaz constitué de dioxyde de carbone dans différentes conditions bien déterminées de concentration et de pressions où le dioxyde de carbone joue un rôle de diluat et permet d'améliorer la selectivité de la réaction.

Pour une grande majorité de procédés d'oxydation, la sélectivité en produit recherché et le taux de conversion peuvent être améliorées par augmentation de la concentration en oxygène dans le mélange réactionnel. Cette concentration est limitée soit par des problèmes technologiques d'évacuation de la chaleur de réaction soit par le domaine d'inflammabilité du mélange à l'entrée ou à la sortie du réacteur ainsi qu'en tout autre point de la section réactionnelle. En effet, tout mélange de réactif combustible et d'oxygène peut s'enflammer lorsque sa teneur en oxygène dépasse une certaine valeur limite, propre à chaque mélange réactionnel et aux conditions de pression et de température mises en oeuvre. Dans les conditions de mise en oeuvre de ce type de réaction, il convient donc de ne pas dépasser cette valeur limite en oxygène admissible en tout point de la section réactionnelle d'une unité de production où la réaction d'oxydation est mise en oeuvre et en particulier dans le mélange réactionnel, notamment pour des raisons de sécurité.

Il serait pourtant particulièrement interessant, pour un mélange d'un même réactif et d'oxygène, dans des conditions de pression et de températures similaires, d'améliorer l'évacuation de la chaleur de réaction et d'augmenter la concentration en oxygène admissible dans le mélange réactionnel sans pour autant, bien entendu, l'enflammer.

Le brevet allemand 1.254.137 décrit un procédé de fabrication d'oxyde d'éthylène par oxydation de l'éthylène, selon lequel on introduit dans le mélange réactionnel du méthane en vue d'augmenter la concentration en oxygène dans la zone réactionnelle. Cependant, ce brevet allemand ne concerne que, strictement, l'oxydation de l'éthylène en présence de méthane en vue de l'obtention d'oxyde d'éthylène. Il est particulièrement notable que l'éthane est considéré dans le cadre de cette réaction d'oxydation, comme ayant un effet contraire à celui du méthane.

Par ailleurs, il est indiqué dans ce même brevet allemand que jusqu'alors, on considérait comme essentiel que la concentration des hydrocarbures paraffiniques dans le circuit, et par suite aussi dans l'alimentation en éthylène, soit réduite à un minimum.

Ceci permet d'expliquer pourquoi, depuis 1962, aucune autre réaction d'oxydation, que celle décrite dans ledit brevet allemand n'ait été mise en oeuvre en présence de méthane ou d'un autre hydrocarbure paraffinique.

En effet, si l'on peut considérer que dans le cadre de l'oxydation d'un alcène, essentiellement l'éthylène, en vue de l'obtention d'un oxyde d'alcène tel l'oxyde d'éthylène, le méthane, contrairement à l'éthane, permet d'augmenter la concentration en oxygène admissible, l'homme du métier n'a aucune raison, au vu dudit brevet allemand, d'utiliser un hydrocarbure paraffinique, réputé néfaste de par sa propension à s'enflammer, en vue d'augmenter la concentration en oxygène d'une autre réaction d'oxydation. Autrement dit, il apparaît, selon l'enseignement de ce brevet allemand, que l'utilisation du méthane en vue d'augmenter la concentration admissible en oxygène ne peut être valable que dans le seul cas de l'oxydation d'un alcène, tel l'éthylène, en vue de l'obtention d'un oxyde d'alcène, tel l'oxyde d'éthylène.

Le brevet allemand n'a pas apporté de solution à l'un des problèmes posés ci-dessus, à savoir augmenter la concentration en oxygène admissible dans un mélange réactionnel en vue d'augmenter la productivité de toute réaction d'oxydation ménagée autre que l'oxydation d'un alcène en vue de l'obtention d'un oxyde d'alcène.

La présente invention concerne alors un procédé permettant d'améliorer la sélectivité d'une réaction d'oxydation ménagée. Un deuxième objet de l'invention consiste à permettre d'augmenter la concentration en oxygène admissible dans le mélange réactionnel et cela, sans problème de sécurité.

Un troisième objet de l'invention consiste en la mise en oeuvre d'un mélange réactionnel dont les propriétés thermiques notamment la conductibilité thermique et la chaleur spécifique, sont améliorées, permettant ainsi un meilleur contrôle de la température de la réaction d'oxydation, ainsi qu'une évacuation de la chaleur de réaction plus aisée au niveau du réacteur.

Un autre objet de l'invention consiste à améliorer la capacité ou le rendement d'une unité de production pour la mise en oeuvre d'une reaction d'oxydation ménagée.

La présente invention concerne donc un procédé pour améliorer une réaction d'oxydation ménagée entre au moins un composé organique et de l'oxygene ou un gaz contenant de l'oxygène, formant un mélange réactionnel, caractérisé en ce qu'on fait réagir au moins un composé organique avec de l'oxygène en présence d'au moins un tiers gaz introduit dans ledit mélange réactionnel et choisi parmi le méthane, l'éthane, et l'hélium, puis, le cas échéant, on traite le produit résultant de la réaction d'oxydation en vue de l'obtention d'un produit final, la réaction d'oxydation d'un alcène par l'oxygène en vue de l'obtention d'un oxyde d'alcène étant exclue.

D'autres caractéristiques et avantages de la présente invention sont à présent décrits.

La figure 1 représente le diagramme ternaire du mélange réactionnel mis en oeuvre dans le procédé d'oxydation du propylène en vue de l'obtention d'acide acrylique en présence de différents tiers gaz ou d'azote.

La figure 2 représente le diagramme ternaire du mélange réactionnel mis en oeuvre dans le procédé d'oxydation du n-butane en vue de l'obtention d'anhydride maléique en présence de différents tiers gaz ou d'azote.

Habituellement, la teneur molaire du tiers gaz introduit dans le mélange réactionnel, constitué par le ou les réactifs et l'oxygène mis en oeuvre, est comprise entre 5 et 80 %, de préférence entre 10 et 70 % et plus généralement entre 20 et 60 %.

L'oxygène mis en oeuvre dans la réaction d'oxydation ménagée, selon le procédé de l'invention peut être de l'oxygène pur ou un gaz contenant de l'oxygène et un gaz inerte vis à vis de ladite réaction d'oxydation ménagée.

Un tel gaz contenant de l'oxygène est le plus souvent de l'air ou de l'air enrichi en oxygène. On comprend alors qu'outre le tiers gaz selon l'invention, le mélange réactionnel peut comporter un ou plusieurs gaz inertes tel l'azote.

Selon la présente invention, on signifie par l'expression "un tiers gaz introduit dans le mélange réactionnel" que ledit tiers gaz est effectivement introduit dans le mélange réactionnel constitué, mais aussi que le tiers gaz peut être préalablement mélangé à l'oxygène ou au gaz contenant l'oxygène, ou, de préférence au(x) réactif(s), destiné(s) à former le mélange réactionnel.

Le tiers gaz peut également être issu d'une réaction d'oxydation ménagée mise en oeuvre préalablement selon le procédé de l'invention, et que l'on recycle en vue d'une nouvelle opération, le cas échéant après traitement et/ou séparation, par adsorption et/ou absorption, et/ou distillation, et/ou condensation fractionnée et/ou perméation. On souligne ici encore le caractère surprenant que constitue l'emploi de gaz tels, en particulier, le méthane ou l'éthane en tant que tiers gaz inerte vis-à-vis d'une réaction d'oxydation ménagée, ces gaz étant habituellement reputés pour leur capacité à s'enflammer. Ceci est d'autant plus surprenant que, dans le cadre de la présente invention, le méthane et, dans une moindre mesure l'éthane, sont des tiers gaz préférés en vue d'augmenter la concentration en oxygène dans le mélange réactionnel, cela de manière plus importante encore qu'avec l'hélium ou le gaz carbonique réputés inertes.

C'est un des mérites de la présente invention d'avoir mis en avant de telles propriétés de ces gaz dans d'autres réactions d'oxydation ménagée que celles de l'oxydation d'alcène par l'oxygène en présence de méthane en vue de préparer un oxyde d'alcène, ce type de réaction devant être considérée jusqu'alors comme un cas particulier ne pouvant être adaptée à d'autres réactions d'oxydation ménagée.

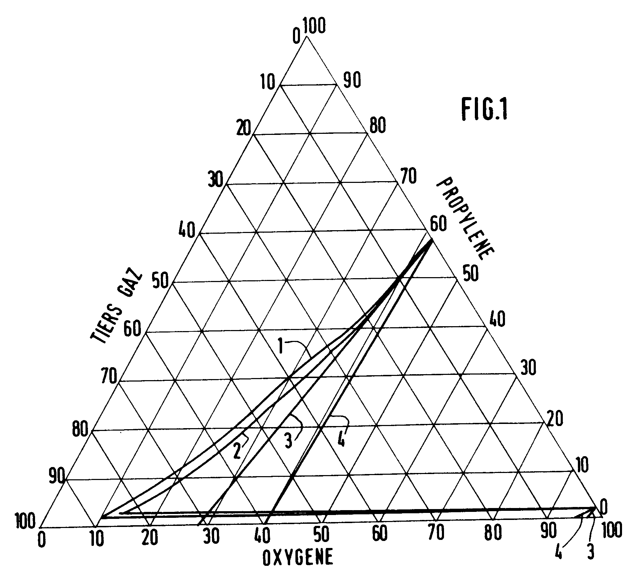

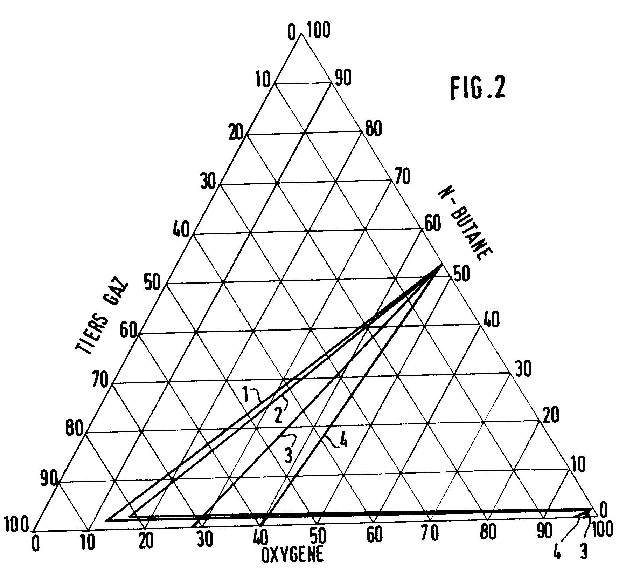

Cette possibilité d'améliorer les réactions d'oxydation ménagée par mise en oeuvre d'un tiers gaz inerte a pu être mise en avant à partir de résultats expérimentaux sur la base desquels ont été tracés des diagrammes ternaires de réactions d'oxydation ménagée mises en oeuvre en présence de différents tiers gaz et dans des conditions de pression et de température habituellement utilisées pour ce type de réaction. Il a ainsi pu être constaté que quelque soit la nature de la réaction d'oxydation ménagée, la teneur en oxygène admissible dans le mélange réactionnel est toujours augmentée lorsqu'on met en oeuvre un tiers gaz choisi parmi le méthane, l'éthane, l'hélium ou le gaz carbonique, par rapport à un autre gaz, habituellement l'azote. Les figures 1 et 2 montrent ces résultats dans le cadre, respectivement, de l'oxydation du propylène en vue de la production d'acide acrylique et de l'oxydation du n-butane en vue de la production d'anhydride maléique, réalisées dans des conditions de températures et de pression habituelles. Pour chacune de ces figures, les courbes 1, 2, 3 et 4 correspondent, respectivement, à l'utilisation d'azote, de gaz carbonique, d'éthane et de méthane en tant que tiers gaz.

Les tracés de ces courbes ont été obtenus expérimentalement selon la méthode classique en elle-même décrite ci-après.

Dans un récipient en acier inoxydable, muni d'un dispositif d'inflammation, on introduit en des teneurs prédéterminées le ou les réactifs et le tiers gaz. Ces composés sont homogénéisés par un dispositif d'agitation.

On introduit alors dans le récipient des teneurs croissantes en oxygène jusqu'à explosion du mélange réactionnel. La teneur en oxygène à laquelle l'explosion se produit est reportée sur un diagramme, en fonction de la teneur en réactif employée.

L'opération est répétée plusieurs fois avec des teneurs variables en réactif, de sorte à permettre l'obtention de plusieurs points sur le diagramme ternaire.

Le procédé selon l'invention permet, selon la nature de la réaction d'oxydation de mettre en oeuvre une teneur admissible en oxygène dans le mélange réactionnel qui peut être supérieure de 1 à 40% molaire, plus généralement de 1 à 20 % molaire (% absolu dans le mélange réactionnel), à la teneur en oxygène admissible lorsque l'on n'introduit pas dans ledit mélange réactionnel un tiers gaz selon l'invention.

Les réactions d'oxydation selon le procédé de l'invention peuvent être mises en oeuvre à la pression atmosphérique, mais aussi à une pression supérieure à 0,1 MPa, de préférence à une pression comprise entre 0,2 et 5 MPa, plus préférentiellement entre 0,2 et 1,5 MPa.

Selon une variante préférée de l'invention, le tiers gaz utilisé pour améliorer la réaction d'oxydation ménagée est le méthane ou un mélange de méthane avec l'éthane, le gaz carbonique et/ou l'hélium.

Outre leur intérêt en vue d'augmenter la concentration en oxygène admissible dans le mélange réactionnel mis en oeuvre, les tiers gaz selon l'invention, peuvent aussi, de par leur conductibilité thermique et leur chaleur spécifique être utilisés pour améliorer fortement la conductibilité thermique et la chaleur spécifique globale du mélange réactionnel ce qui facilite l'évacuation de la chaleur pour une même quantité de charge traitée.

Dans la mesure où l'évacuation de la chaleur du mélange réactionnel est améliorée, il est alors possible d'augmenter soit la capacité soit le rendement d'une unité de production pour la mise en oeuvre d'une réaction d'oxydation ménagée. Un autre objet de l'invention consiste alors en l'utilisation d'au moins un tiers gaz choisi parmi le méthane, l'éthane, le gaz carbonique et l'hélium en vue d'améliorer l'évacuation de la chaleur de réaction produite lors d'une réaction d'oxydation ménagée, la réaction d'oxydation d'un alcène par l'oxygène en vue de l'obtention d'un oxyde d'alcène étant exclue.

Pour une mise en oeuvre du procédé selon l'invention, plus particulièrement destinée à améliorer la conductibilité thermique et la chaleur spécifique globale du mélange réactionnel, le tiers gaz est de préférence l'hélium ou le méthane.

L'utilisation d'un tiers gaz selon l'invention, notamment en vue d'améliorer l'évacuation de la chaleur de réaction, est plus particulièrement adapté aux réactions d'oxydation effectuées en lit fixe.

Le procédé selon l'invention peut être mis en oeuvre pour améliorer toute réaction d'oxydation ménagée, qu'elle soit réalisée en phase gazeuse ou en phase gaz/liquide.

Le procédé selon l'invention est tout particulièrement adapté aux réactions d'oxydation ménagée suivantes :

- oxydation du cumène en vue de préparer le phénol ;
- oxychloration de l'éthylène en dichloroéthane ;
- oxychloration du benzène en vue de préparer le phénol ;
- oxydation du toluène en vue de préparer l'acide benzoïque ou le phénol;
- oxydation de l'isobutane ou de l'éthylbenzène en vue de l'obtention d'oxyde de propylène ;
- oxydation en phase liquide de l'éthylène en vue de l'obtention d'acétaldéhyde ;
- oxydation de l'acétaldéhyde, du n-butane, d'essences légères (à majorité coupe $C_5$ - $C_6$) ou de n-butènes en vue de l'obtention d'acide acétique ;
- oxydation du propylène ou de l'isopropanol en vue de l'obtention d'acétone ;
- oxydation de l'éthylène en présence d'acide acétique en vue de l'obtention d'acétate de vinyle ;
- oxydation du butadiène en présence d'acide acétique en vue de l'obtention de butanediol-1,4 ;
- oxydation du propylène en vue de l'obtention d'acide acrylique ;
- oxydation de l'alcool butylique tertiaire en vue de l'obtention d'acide méthacrylique ;
- oxydation du cyclohexane en vue de l'obtention de caprolactame et d'acide adipique ;
- oxydation du p-xylène, du toluène ou de l'aldéhyde p-toluique en vue de l'obtention d'acide téréphtalique ;
- oxydation du p-xylène en vue de l'obtention du téréphtalate de diméthyle ;
- oxydation du benzène, de n-butènes ou du n-butane en vue de l'obtention d'anhydride maléique ;
- oxydation de l'orthoxylène ou du naphtalène en vue de l'obtention d'anhydride phtalique ;

- oxydation du méthanol en vue de l'obtention de formol.
- oxydation du glyoxal en vue de l'obtention de glycol.
- oxydation d'aldéhyde en vue de l'obtention d'anhydrides ou d'acides carboxyliques.

Les exemples suivants ont pour but d'illustrer la présente invention :

EXEMPLE 1

En vue de déterminer la teneur en oxygène maximale admissible dans un mélange constitué de propylène et d'oxygène, et mis en oeuvre industriellement pour produire de l'acide acrylique, on procède comme suit :

On introduit des teneurs variables de propylène et de tiers gaz ou d'azote dans un récipient en acier inoxydable muni d'un dispositif d'inflammation. On homogénéise les mélanges ainsi réalisés et on y introduit des teneurs croissantes en oxygène jusqu'à ce que le mélange s'enflamme.

Les conditions de pression et température sont celles habituellement mises en oeuvre dans l'industrie pour réaliser cette réaction d'oxydation ménagée.

La teneur en oxygène maximale admissible, au-delà de laquelle le mélange s'enflamme est reportée dans le Tableau I ci-après.

| Propylène (% mol.) | Oxygène (% mol.) | tiers gaz ou azote (% mol.) | Nature du tiers gaz |
|---|---|---|---|
| 6,8 | 15 | 78,2 | azote |
| 15,5 | 22 | 62,5 | |
| 6,6 | 18 | 75,4 | $CO_2$ |
| 15,3 | 24 | 60,7 | |
| 8 | 40,3 | 51,7 | méthane |
| 28 | 41 | 31 | |
| 4 | 29 | 67 | éthane |
| 13,6 | 32 | 54,4 | |

Au vu du Tableau I, il apparaît clairement que la teneur en oxygène dans le mélange est nettement augmentée lorsque l'on y introduit un tiers gaz selon l'invention par rapport à la teneur en oxygène admissible quand seul de l'azote est introduit dans le mélange.

EXEMPLE 2

On procède comme dans l'exemple 1 en vue de déterminer la teneur en oxygène maximale admissible dans un mélange constitué de n-butane et d'oxygène, mis en oeuvre industriellement pour produire de l'anhydride maléique.

Les conditions de pression et de température sont celles habituellement mises en oeuvre dans l'industrie pour réaliser cette réaction d'oxydation ménagée.

La teneur en oxygène maximale admissible au-delà de laquelle le mélange s'enflamme est reportée dans le Tableau II ci-après :

| n-butane (% mol.) | oxygène (% mol.) | tiers gaz ou azote (% mol.) | nature du tiers gaz |
|---|---|---|---|
| 2,15 | 13 | 84,85 | azote |
| 4,3 | 14 | 82,70 | |
| 8,3 | 17 | 74,70 | |
| 4,15 | 17 | 78,85 | $CO_2$ |
| 8,1 | 19 | 72,90 | |
| 5 | 41 | 54 | méthane |
| 10 | 41,8 | 48,20 | |
| 5 | 30 | 75 | éthane |
| 10 | 32 | 58 | |

Ces résultats, comme ceux figurant dans l'exemple 1, montrent que l'introduction dans un mélange de méthane, d'éthane ou de gaz carbonique permet d'augmenter considérablement la teneur maximale admissible d'oxygène.

## EXEMPLE 3

Oxychloration à l'air de l'éthylène en lit fixe

Cette réaction est réalisée dans trois réacteurs disposés en série et opérant en lit fixe. Chaque réacteur comporte à titre de catalyseur des chlorures de cuivre et de potassium supportés par des billes d'alumine de 3 mm de diamètre.
Le premier réacteur est alimenté par les composés suivants :

| | % mol. |
|---|---|
| azote | 30 |
| oxygène | 8,8 |
| éthylène | 21 |
| acide chlorhydrique | 36,9 |
| hélium | 3,3 |

L'oxygène introduit dans le premier réacteur représente 80 % de la totalité de l'oxygène utilisé dans le procédé.
La réaction d'oxychloration est effectuée dans chacun de trois réacteurs à une température moyenne de 214°C et à une pression de 6,25 bar absolu.
Le mélange de corps résultant de la réaction d'oxychloration du premier réacteur passe dans le deuxième réacteur, lequel est alimenté par 12 % de l'oxygène total utilisé, puis dans le troisième réacteur alimenté par les 8 % restant de l'oxygène utilisé.
A la sortie du troisième réacteur, on récupère un débit de dichloroéthane de 443 kilomoles/h.
A titre comparatif, on effectue une réaction d'oxychloration dans les mêmes conditions que ci-dessus, mais ou l'hélium est remplacé par de l'azote. A la sortie du troisième réacteur, on récupère un débit de dichloroéthane de 432 kilomoles/h.
On constate donc que l'utilisation d'hélium permet d'augmenter la capacité de traitement de l'unité de 2,5 % ce qui est considérable à l'échelle industrielle. Cette augmentation est due au fait que l'hélium permet une meilleure évacuation de la chaleur du mélange réactionnel. Ainsi, il a pu être noté une diminution de 30° C de la température de la zone chaude dans le premier réacteur quand l'hélium était utilisé à la place de l'azote.

**Revendications**

1. Procédé pour améliorer une réaction d'oxydation ménagée entre au moins un composé organique et de l'oxygène, caractérisé en ce qu'on fait réagir au moins un composé organique avec de l'oxygène ou un gaz contenant de l'oxygène, formant un mélange réactionnel, en présence d'au moins un tiers gaz introduit dans ledit mélange réactionnel et choisi parmi le méthane, l'éthane et l'hélium, puis, le cas échéant, on traite le produit résultant de la réaction d'oxydation en vue de l'obtention d'un produit final, la réaction d'oxydation d'un alcène par l'oxygène en vue de l'obtention d'un oxyde d'alcène étant exclue.

2. Procédé selon la revendication 1, caractérisé en ce que la teneur en mole du tiers gaz dans le mélange réactionnel est comprise entre 5 et 80 %, de préférence entre 10 et 70 % et généralement entre 20 et 60 %.

3. Procédé selon l'une des revendications 1 et 2, caractérisé en ce qu'on effectue l'oxydation à une pression supérieure ou égale à 0,1 MPa, de préférence comprise entre 0,2 et 5 MPa, et plus préférentiellement entre 0,2 et 1,5 MPa.

4. Procédé selon l'une des revendications 1 à 3, caractérisé en ce que le tiers gaz est le méthane ou un mélange de méthane avec l'éthane, le gaz carbonique et/ou l'hélium.

5. Procédé selon l'une des revendications 1 à 4, caractérisé en ce que la teneur en oxygène admissible dans le mélange réactionnel est supérieure de 1 à 40 % molaire, plus généralement de 1 à 20 % molaire (% absolu dans le mélange réactionnel), à la teneur en oxygène admissible dans un même mélange réactionnel dans lequel on n'a pas introduit un tiers gaz tel que défini dans la revendication 1.

6. Procédé selon l'une des revendications 1 à 5, caractérisé en ce qu'on mélange le tiers gaz soit avec l'oxygène ou le gaz contenant l'oxygène, soit, de préférence, avec le ou les composé(s) organique(s) destiné(s) à former le mélange réactionnel.

7. Procédé selon l'une des revendications 1 à 6, caractérisé en ce que le tiers gaz introduit dans le mélange réactionnel est issu d'une réaction d'oxydation ménagée mise en oeuvre selon le procédé d'une des revendications 1 à 5, et que l'on recycle, le cas échéant après traitement et/ou séparation.

8. Procédé selon la revendication 7, caractérisé en ce que ledit traitement et/ou ladite séparation est une ou plusieurs des opérations consistant en une adsorption, une absorption, une distillation, une condensation fractionnée ou une perméation.

9. Procédé selon l'une des revendications 1 à 8, caractérisé en ce que la réaction d'oxydation ménagée mise en oeuvre consiste en l'une des réactions suivantes :

   - oxydation du cumène en vue de préparer le phénol ;
   - oxychloration de l'éthylène en dichloroéthane ;
   - oxychloration du benzène en vue de préparer le phénol ;
   - oxydation du toluène en vue de préparer l'acide benzoïque ou le phénol;
   - oxydation de l'isobutane ou de l'éthylbenzène en vue de l'obtention d'oxyde de propylène ;
   - oxydation en phase liquide de l'éthylène en vue de l'obtention d'acétaldéhyde ;
   - oxydation de l'acétaldéhyde, du n-butane, d'essences légères (à majorité coupe $C_5$-$C_6$) ou de n-butènes en vue de l'obtention d'acide acétique ;
   - oxydation du propylène ou de l'isopropanol en vue de l'obtention d'acétone ;
   - oxydation de l'éthylène en présence d'acide acétique en vue de l'obtention d'acétate de vinyle ;
   - oxydation du butadiène en présence d'acide acétique en vue de l'obtention de butanediol-1,4 ;
   - oxydation du propylène en vue de l'obtention d'acide acrylique ;
   - oxydation de l'alcool butylique tertiaire en vue de l'obtention d'acide méthacrylique ;
   - oxydation du cyclohexane en vue de l'obtention de caprolactame et d'acide adipique ;
   - oxydation du p-xylène, du toluène ou de l'aldéhyde p-toluique en vue de l'obtention d'acide téréphtalique ;
   - oxydation du p-xylène en vue de l'obtention du téréphtalate de diméthyle ;
   - oxydation du benzène, de n-butènes ou du n-butane en vue de l'obtention d'anhydride maléique ;
   - oxydation de l'orthoxylène ou du naphtalène en vue de l'obtention d'anhydride phtalique ;
   - oxydation du méthanol en vue de l'obtention de formol.
   - oxydation du glyoxal en vue de l'obtention de glycol.

- oxydation d'aldéhydes en vue de l'obtention d'anhydrides ou d'acides carboxyliques.

**10.** Utilisation d'au moins un tiers gaz choisi parmi le méthane, l'éthane, le gaz carbonique et l'hélium en vue d'augmenter la teneur en oxygène admissible d'une réaction d'oxydation ménagée d'au moins un composé organique par l'oxygène, la réaction d'oxydation d'un alcène par l'oxygène en vue de l'obtention d'un oxyde d'alcène étant exclue.

**11.** Utilisation selon la revendication 10, caractérisée en ce que ledit tiers gaz est le méthane ou un mélange de méthane avec l'éthane, le gaz carbonique et/ou l'hélium.

**12.** Utilisation d'au moins un tiers gaz choisi parmi le méthane, l'éthane, le gaz carbonique et l'hélium en vue d'améliorer l'évacuation de la chaleur de réaction produite lors d'une réaction d'oxydation ménagée d'au moins un composé organique par l'oxygène, la réaction d'oxydation d'un alcène par l'oxygène en vue de l'obtention d'un oxyde d'alcène étant exclue.

**13.** Utilisation selon la revendication 12, caractérisée en ce que ledit tiers gaz est l'hélium et/ou le méthane.

## Claims

1. A method of improving a smooth oxidation reaction between at least one organic compound and oxygen, characterised in that a reaction is made between at least one organic compound and oxygen or a gas containing oxygen, forming a reaction mixture, in the presence of at least a third gas introduced into the said reaction mixture and selected from methane, ethane and helium, then if need be, the product resulting from the oxidation reaction is treated with a view of obtaining a final product, the oxidation reaction of an alkene by oxygen with a view of obtaining an alkene oxide being excluded.

2. A method according to claim 1, characterised in that the molar content of the third gas in the reaction mixture is comprised between 5 and 80%, of preference between 10 and 70% and generally between 20 and 60%.

3. A method according to one of claims 1 and 2, characterised in that the oxidation is carried out at a pressure greater than or equal to 0.1 MPa, of preference comprised between 0.2 and 5 MPa, and more preferentially between 0.2 and 1.5 MPa.

4. A method according to one of claims 1 to 3, characterised in that the third gas is methane or a mixture of methane with ethane, carbon dioxide and/or helium.

5. A method according to one of claims 1 to 4, characterised in that the acceptable content of oxygen in the reaction mixture is greater than 1 to 40 molar %, more generally from 1 to 20 molar % (absolute % in the reaction mixture), of the acceptable content of oxygen in the same reaction mixture in which a third gas such as defined in claim 1 has not been introduced.

6. A method according to one of claims 1 to 5, characterised in that the third gas is mixed with either oxygen or a gas containing oxygen, or, of preference, with the organic compound(s) destined to form the reaction medium.

7. A method according to one of claims 1 to 6, characterised in that the third gas introduced into the reaction mixture results from a smooth oxidation reaction carried out according to the process of one of claims 1 to 5, and that one recycles, if need be after treatment and/or separation.

8. A method according to claim 7, characterised in that the said treatment and/or the said separation is one or more operations consisting in an adsorption, an absorption, a distillation, a fractionated condensation or a permeation.

9. A method according to claims 1 to 8, characterised in that the smooth oxidation reaction carried out consists in one of the following reactions:

    - an oxidation of cumene with a view to preparing phenol;
    - an oxychlorination of ethylene to give dichoroethane;
    - an oxychlorination of benzene with a view to preparing phenol;
    - an oxidation of toluene with a view to preparing benzoic acid or phenol;
    - an oxidation of isobutane or ethyl benzene with a view to obtaining propylene oxide;

- a liquid phase oxidation of ethylene with a view of obtaining acetaldehyde;
- an oxidation of acetaldehyde, n-butane or light petrols (of majority $C_5$-$C_6$ range) or n-butenes with a view of obtaining acetic acid;
- an oxidation of propylene or isopropanol with a view of obtaining acetone;
- an oxidation of ethylene in the presence of acetic acid with a view of obtaining vinyl acetate;
- an oxidation of butadiene in the presence of acetic acid with a view of obtaining buta-1,4-diol;
- an oxidation fo propylene with a view of obtaining acrylic acid;
- an oxidation of tertiary butyl alcohol with a view of obtaining methacrylic acid;
- an oxidation of cyclohexane with a view of obtaining caprolactam and adipic acid;
- an oxidation of p-xylene, toluene or p-toluic aldehyde with a view of obtaining terephthalic acid;
- an oxidation of p-xylene with a view of obtaining dimethyl terephthalate;
- an oxidation of benzene, n-butenes or n-butane with a view of obtaining maleic anhydride;
- an oxidation of ortho-xylene or naphthalene with a view of obtaining phthalic anhydride;
- an oxidation of methanol with a view of obtaining formol.
- an oxidation of glyoxal with a view of obtaining glycol.
- an oxidation of aldehydes with a view of obtaining carboxylic anhydrides or acids.

10. Use of at least one third gas selected from methane, ethane, carbon dioxide and helium with a view of increasing the acceptable content of oxygen in a smooth oxidation reaction of at least one organic compound with oxygen, the oxidation reaction of an alkene with oxygen with a view of obtaining an alkene oxide being excluded.

11. Use according to claim 10, characterised in that the said third gas is methane or a mixture of methane and ethane, carbon dioxide and/or helium.

12. Use of at least one third gas selected from methane, ethane, carbon dioxide and helium with a view of improving the evacuation of the reaction heat produced during a smooth oxidation reaction of at least one organic compound with oxygen, the oxidation reaction of an alkene with oxygen with a view of obtaining an alkene oxide being excluded.

13. Use according to claim 12, characterised in that the said third gas is helium and/or methane.

**Patentansprüche**

1. Verfahren zur Verbesserung einer gelenkten Oxidationsreaktion zwischen zumindest einer organischen Verbindung und Sauerstoff, dadurch gekennzeichnet, daß zumindest eine organische Verbindung mit Sauerstoff oder einem sauerstoffhaltigen Gas zur Bildung einer Reaktionsmischung in Anwesenheit zumindest eines dritten Gases umgesetzt wird, das in die Reaktionsmischung eingebracht und aus Methan, Ethan und Helium ausgewählt wird, und dann gegebenenfalls das aus der Oxidationsreaktion erhaltene Produkt zum Erhalten eines Endprodukts behandelt wird, wobei die Oxidationsreaktion eines Alkens durch Sauerstoff zum Erhalten eines Alkenoxids ausgeschlossen ist.

2. Verfahren nach Anspruch 1, dadurch gekennzeichnet, daß der Molgehalt des dritten Gases in der Reaktionsmischung zwischen 5 und 80 %, vorzugsweise zwischen 10 und 70 % und allgemein zwischen 20 und 60 % liegt.

3. Verfahren nach einem der Ansprüche 1 und 2, dadurch gekennzeichnet, daß die Oxidation bei einem Druck von größer oder gleich 0,1 MPa, vorzugsweise zwischen 0,2 und 5 MPa und bevorzugter zwischen 0,2 und 1,5 MPa durchgeführt wird.

4. Verfahren nach einem der Ansprüche 1 bis 3, dadurch gekennzeichnet, daß das dritte Gas Methan oder eine Mischung von Methan mit Ethan, Kohlendioxid und/oder Helium ist.

5. Verfahren nach einem der Ansprüche 1 bis 4, dadurch gekennzeichnet, daß der zulässige Sauerstoffgehalt in der Reaktionsmischung mehr als 1 bis 40 Mol-%, allgemeiner 1 bis 20 Mol-% (absoluter Prozentsatz in der Reaktionsmischung), des zulässigen Sauerstoffgehalts einer gleichen Reaktionsmischung beträgt, in die kein drittes Gas, wie in Anspruch 1 definiert, eingebracht wurde.

6. Verfahren nach einem der Ansprüche 1 bis 5, dadurch gekennzeichnet, daß das dritte Gas entweder mit Sauerstoff oder dem sauerstoffhaltigen Gas, oder vorzugsweise mit der bzw. den zur Bildung der Reaktionsmischung bestimmten organischen Verbindung bzw. Verbindungen gemischt wird.

7. Verfahren nach einem der Ansprüche 1 bis 6, dadurch gekennzeichnet, daß das dritte Gas, das in die Reaktionsmischung eingebracht wird, von einer gelenkten Oxidationsreaktion stammt, die gemäß dem Verfahren nach einem der Ansprüche 1 bis 5 durchgeführt wurde, und daß es gegebenenfalls nach Behandlung und/oder Trennung rückgeführt wird.

8. Verfahren nach Anspruch 7, dadurch gekennzeichnet, daß die Behandlung und/oder Trennung eine oder mehrere Vorgänge sind, die aus einer Adsorption, einer Absorption, einer Destillation, einer fraktionierten Kondensation oder einer Permeation bestehen.

9. Verfahren nach einem der Ansprüche 1 bis 8, dadurch gekennzeichnet, daß die durchgeführte gelenkte Oxidationsreaktion aus einer der folgenden Reaktionen besteht:

- Oxidation von Cumol zur Herstellung von Phenol;
- Oxychlorierung von Ethylen zu Dichlorethan;
- Oxychlorierung von Benzol zur Herstellung von Phenol;
- Oxidation von Toluol zur Herstellung von Benzoesäure oder Phenol;
- Oxidation von Isobutan oder Ethylbenzol zum Erhalten von Propylenoxid;
- Flüssigphasen-Oxidation von Ethylen zum Erhalten von Acetaldehyd;
- Oxidation von Acetaldehyd, n-Butan, Leichtbenzin (hauptsächlich $C_5$-$C_6$-Fraktion) oder n-Butenen zum Erhalten von Essigsäure;
- Oxidation von Propylen oder Isopropanol zum Erhalten von Aceton;
- Oxidation von Ethylen in Anwesenheit von Essigsäure zum Erhalten von Vinylacetat;
- Oxidation von Butadien in Anwesenheit von Essigsäure zum Erhalten von 1,4-Butandiol;
- Oxidation von Propylen zum Erhalten von Acrylsäure;
- Oxidation von tert.Butylalkohol zum Erhalten von Methacrylsäure;
- Oxidation von Cyclohexan zum Erhalten von Caprolactam und Adipinsäure;
- Oxidation von p-Xylol, Toluol oder p-Tolylaldehyd zum Erhalten von Terephthalsäure;
- Oxidation von p-Xylol zum Erhalten von Dimethylterephthalat;
- Oxidation von Benzol, n-Butenen oder von n-Butan zum Erhalten von Maleinsäureanhydrid;
- Oxidation von Orthoxylol oder Naphthalin zum Erhalten von Phthalsäureanhydrid;
- Oxidation von Methanol zum Erhalten von Formol;
- Oxidation von Glyoxal zum Erhalten von Glykol;
- Oxidation von Aldehyden zum Erhalten von Carbonsäureanhydriden.

10. Verwendung zumindest eines dritten Gases, ausgewählt aus Methan, Ethan, Kohlendioxid und Helium, zur Erhöhung des zulässigen Sauerstoffgehalts einer gelenkten Oxidationsreaktion zumindest einer organischen Verbindung durch Sauerstoff, wobei die Oxidationsreaktion eines Alkens durch Sauerstoff zum Erhalten eines Alkenoxids ausgeschlossen ist.

11. Verwendung nach Anspruch 10, dadurch gekennzeichnet, daß das dritte Gas Methan oder eine Mischung von Methan mit Ethan, Kohlendioxid und/oder Helium ist.

12. Verwendung zumindest eines dritten Gases, ausgewählt aus Methan, Ethan, Kohlendioxid und Helium, zur Verbesserung der Abfuhr der Reaktionswärme, die bei einer gelenkten Oxidationsreaktion zumindest einer organischen Verbindung durch Sauerstoff erzeugt wird, wobei die Oxidationsreaktion eines Alkens durch Sauerstoff zum Erhalten eines Alkenoxids ausgeschlossen ist.

13. Verwendung nach Anspruch 12, dadurch gekennzeichnet, daß das dritte Gas Helium und/oder Methan ist.

FIG.1

FIG.2

EP 0 558 415 B1